# EUROPÄISCHE PATENTSCHRIFT

(11) **EP 0 826 370 B1**
(45) Veröffentlichungstag und Bekanntmachung des Hinweises auf die Patenterteilung: **19.06.2002**
(21) Anmeldenummer: 97104503.4
(22) Anmeldetag: 17.03.1997
(51) Int. Cl.: A61K 31/41, A61K 33/04, A61P 25/28

(54) **Pharmazeutische Zubereitung, die 2-Phenyl-1,2-Benzoisoelenazol-3(2H)-one enthält, für die Behandlung der Alzheimer-Krankheit**
Pharmaceutical composition comprising 2-phenyl-1,2-benzoisoelenazol-3(2H)-one for the treatment of Alzheimer disease
Composition pharmaceutique contenant de la 2-phényl-1,2-benzoisoelenazol-3(2H)-one pour traiter la maladie d'Alzheimer

(30) Priorität: 27.08.1996 JP 22551296
(43) Veröffentlichungstag der Anmeldung: 04.03.1998
(73) Patentinhaber: A. Nattermann & Cie. GmbH, 50829 Köln (DE)
(72) Erfinder: Maruyama, Ikuro, Kagoshima-shi, Kagoshima (JP); Abeyama, Kazuhiro, Kagoshima-shi, Kagoshima (JP); Masayasu, Hiroyuko, c/o Tokyo res. & dev. cent., Edogawa-ku, Tokyo (JP)
(74) Vertreter: Fischer, Hans-Jürgen

(56) Entgegenhaltungen:
- WO-A-92/02221
- DAWSON ET AL: "the neuroprotective efficacy of ebselen on brain damage..." NEUROSCIENCE LETTERS, Bd. 185, Nr. 1, 1995, Seiten 65-69, XP002040434
- KNOLLEMA ET AL: "ebselen (pz-51) protects the caudate futamen against hypoxia/ischemia induced neuronal damage" NEUROSCIENCE RES. COM., Bd. 19, Nr. 1, Juli 1996 - August 1996, Seiten 47-56, XP002040435
- STOHS: "the role of free radicals in toxicity and disease" J OF BASIC AND CLIN. PHYS. AND PHARM., Bd. 6, Nr. 3-4, 1995, Seiten 205-228, XP002040436

## Beschreibung

Die vorliegende Erfindung betrifft eine präventive und/oder therapeutische Verwendung von 2-Phenyl-1,2-Benzisoselenazol-3(2H)-one.

In einigen Ländern werden Acetylcholinsterase-Inhibitoren, so zum Beispiel Physostigmin oder Tetrahydroaminoakridin (THA), als vorbeugende und/oder therapeutische Arzneimittel für die Alzheimer-Krankheit verwendet. Dabei konnten jedoch keine Erfolge verzeichnet werden. Ebenso wurde der Versuch unternommen, 5-HT-Antagonisten, die auf das Nervensystem wirken, zu entwickeln, jedoch ist deren Mechanismus noch nicht vollständig aufgeklärt.

Die Verwendung von 2-Phenyl-1,2-Benzisoselenazol-3(2H)-one, das auch allgemein als Ebselen bezeichnet wird, in pharmazeutischen Zubereitungen ist bekannt. So beschreibt die WO92/02221 A die Verwendung dieses 2-Phenyl-1,2-Benzisoselenazol-3(2H)-one in pharmazeutischen Zubereitungen zur Behandlung von solchen Nebenwirkungen, die in der Tumortherapie durch Cisplatin verursacht werden. Desweiteren sind den Veröffentlichungen von Dawson et al in Neuroscience Letters, 185 (1995), S. 65 ff. sowie der Veröffentlichung von Knollema et al in Neuroscience Research Communication, Vol. 19, No. 1, S. 47 zu entnehmen, daß die Gabe von Ebselen in bestimmten Konzentrationen eine neuroschützende Wirksamkeit bei akuten und begrenzten Ischämien besitzt, so daß die hierdurch verursachten Gehirnschäden und insbesondere der bei einem Sauerstoffmangel im Blut auftretende Schlaganfall vermieden werden.

Desweiteren wird in der Veröffentlichung von Stohs in Journal of basis and clinical physiology & pharmacology, Vol. 6, No. 3-4, 1995, S. 218 und 219 die Vermutung geäußert, daß die Alzheimerkrankheit im Zusammenhang mit Sauerstoffradikalen steht.

Die pathologischen Merkmale der Alzheimer-Krankheit umfassen senile Plaques und eine Anhäufung von PHFs (gepaarte schraubenförmige Fasern) im Gehirn.

Das β-Amyloid-Protein, das nachfolgend auch mit Aβ bezeichnet ist und das den Hauptbestandteil der senilen Plaques darstellt, ist ein unlösliches Peptid, bestehend aus etwa 40 Aminosäuren-Resten. Es wurde festgestellt, daß das Aβ seinerseits Neuronen biochemisch schädigt und daß insbesondere geronnenes, nicht gelöstes Aβ diesen Effekt hervorruft.

Somit war die Entwicklung einer Substanz erwünscht, die dem Fortschreiten der Alzheimer-Krankheit vorbeugt und/oder sie heilt, wobei diese Substanz aus einer pharmazeutischen Zubereitung besteht, die das durch toxisches Aβ herbeigeführte Absterben von Neuronen unterdrückt.

Diese Aufgabe wird durch die erfindungsgemäße Verwendung des 2-Phenyl-1,2-Benzisoselenazol-3(2H)-one des Patentanspruchs 1 gelöst.

Die erfindungsgemäße Verwendung berüht auf der überraschenden Erkenntnis, daß 2-Phenyl-1,2-Benzisoselenazol-3(2H)-one imstande ist, die Blutflußgrenze des Gehirns zu überwinden und die Neuronentoxizität des Aβ zu reduzieren. Hierbei wurde überraschend festgestellt, daß das 2-Phenyl-1,2-Benzisoselenazol-3(2H)-one (im folgenden auch kurz als Verbindung A bezeichnet) eine hervorragende Wirkung bei Alzheimer-krankheit aufweist.

Die in der vorliegenden Erfindung verwendete Verbindung A hemmt das durch die Anwesenheit von Aß begründete Absterben von Neuronen. Somit ist die erfindungsgemäße Verwendung der Verbindung A zur Herstellung einer vorbeugende und/oder therapeutische pharmazeutische Zubereitung zur Behandlung der Alzheimer-Krankheit geeignet.

Aß wurde als Hauptbestandteil des senilen Plaques, einem pathologischen Merkmal der Alzheimer-Krankheit, identifiziert. Es besteht aus 39-40 Aminosäure-Resten. Desweiteren ist bekannt, daß das Aβ neurotoxisch wirkt und somit den Zellentod hervorruft. Erst kürzlich wurde herausgefunden, daß das durch das neurotoxische Aβ induzierte Absterben von PC12-Zellen in Gegenwart von Aβ unterdrückt wird, wenn die Verbindung A (Ebselen) hinzugefügt wird.

Die in der vorliegenden Erfindung verwendete Verbindung A kann durch ein in der japanischen Patentschrift (kokoku) Nr. 2-38591 (japanische Offenlegung (kokai) Nr. 57-67568) offenbartes Verfahren synthetisiert werden.

Die unter Verwendung der Verbindung A hergestellte Zuberietung kann in verschiedenen Formen, wie z. B. als Tablette, Kapsel, Puder, Granulat, Sirup und Injektion, durch Anwendung bekannter Herstellungstechniken unter Verwendung von Zusatzstoffen, Trägern, Bindemitteln, Sprengmitteln und Lösungsmitteln dargereicht werden.

Die folgende Tabelle gibt beispielhaft die Zusammensetzung einer Tablette wieder:

| | |
|---|---|
| Verbindung A | 50 mg |
| Carboxymethylcellulose | 25 mg |
| Stärke | 5 mg |
| kristalline Cellulose | 40 mg |
| Magnesiumstearat | 2 mg |
| Gesamt | 122 mg |

Die Verbindung A behält ihre primär beanspruchte Wirkung bei jeder Art der Darreichung, so z. B. bei der üblichen oralen Anwendung oder bei der peroralen Anwendung, beispielsweise bei einer Injektion.

Im Falle der oralen Anwendung liegt die Dosis der Verbindung A für einen Erwachsenen zwischen 100 und 2.000 mg/Tag, vorzugsweise zwischen 200 und 1.000 mg/Tag, wobei diese Dosis abhängig von der klinischen Situation des Patienten entsprechend erhöht oder verringert werden kann.

Bei der Überprüfung der Toxizität der Verbindung A wurden LD₅₀-Werte bei Mäusen und Ratten ermittelt. Gemäß den Versuchen der Erfinder lagen die bei den Mäusen ermittelten LD₅₀-Werte bei ≥ 6.810 mg/kg bei oraler Anwendung und bei 740 mg/kg bei intraperitonealer Anwendung. Die bei Ratten festgestellten LD₅₀-Werte lagen bei ≥ 6.810 mg/kg bei oraler Anwendung und bei 580 mg/kg bei intraperitonealer Anwendung. Diese LD₅₀-Werte beweisen, daß somit die Verbindung A sehr sicher ist.

Selbst dann, wenn eine hohe Dosis der Verbindung A den Mäusen oder Ratten verabreicht wurde, konnten keine problematischen Nebeneffekte beobachtet werden.

Die erfindungsgemäße Verwendung wird nunmehr anhand von Beispielen näher beschrieben.

### Beispiel 1

### Wirkung der Verbindung A als Inhibitor des durch Aβ induzierten Neuronenabsterbens in Anwesenheit von Aβ:

Kultivierte PC12-Zellen (die von Ratten abstammen, Chromaffiner Tumor) wurden in einer 100 mm Schale (von Corning hergestellt) unterkultiviert, die mit Polylysin überzogen ist und die ein DMEM-Medium enthält, welches mit 10 % FCS and 5 % Pferdeserum (Produkt von Sigma) versetzt ist. Mit anderen Worten, die PC12-Zellen (1 x 10⁶ Zellen/Schale) wurden in einer mit Polylysin beschichteten 100 mm Schale angezüchtet. Um Unterschiede der PC12-Zellen aufzuzeigen, wuchsen die PC12-Zellen sieben Tage lang in einem DMEM-Medium, das 5 % FCS, 50 ng/ml NGF und 1 Pferdeserum enthielt, heran. Die PC12-Zellen wurden dann durch Pipettierung abgeschert und in einem geeigneten Medium suspendiert, um so eine Zellsuspension zu erstellen. Die Zellsuspension wurde in jede Vertiefung einer mit Polylysin beschichteten Schale mit 96 Vertiefungen (1 x 10⁴ Zellen/Vertiefung) eingebracht. Hiernach wurde das Aβ (Bachem Feinchemikalien AG) hinzugefügt, so daß eine Endkonzentration von 10 µM erreicht wurde. Die Verbindung A wurde nun ebenfalls hinzugefügt, so daß eine Endkonzentration von 0,1, 1,0 oder 2,5 µM erreicht wurde. Als Kontrollsubstanz wurde eine Verbindung B (Verbindung A substituiert mit Schwefel; keine gluthation-peroxidase-ähnliche Wirkung; 2-Phenyl-1,2-Benzothiazol-3(2H)-one; durch Nattermann zur Verfügung gestellt) in der gleichen Konzentration und auf die gleiche Weise hinzugefügt. Die Zellen wurden für 48 Stunden nach Hinzufügen der Verbindung A oder der Verbindung B kultiviert.

Ein MTT-Reagens (3-(4,5-Dimethylthiazol-2-yl)-2,5-Diphenyl-Tetrazolium-Bromid) wurde den so behandelten Zellen zugesetzt. Die Inkubation wurde für 4 Stunden ausgeführt und die Zellen wurden lysiert. Die nachfolgende Verarbeitung wurde unter Anwendung des sogenannten MTT-Verfahrens durchgeführt, das kolorimetrisch auf der Basis einer Reduktionsreaktion des Reagens arbeitet, um dadurch die Lebensfähigkeit der Zellen in der Suspension (Behl, C. et al., Cell, 77: 817-827, 1994) zu ermitteln.

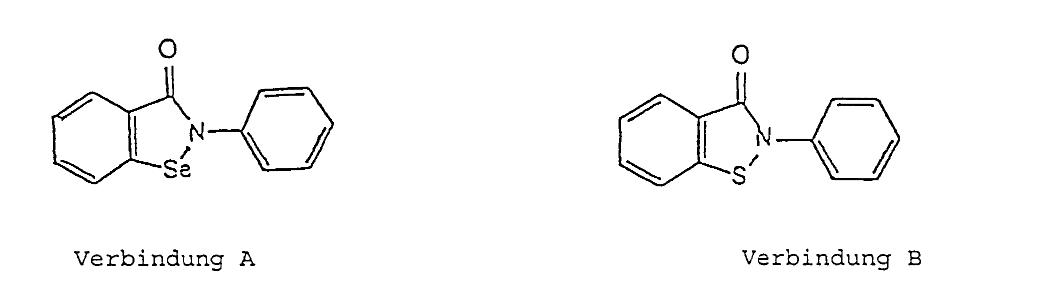

**Tabelle 1:**

| Wirkung der Verbindungen A und B auf das Absterben von Zellen von PC12-Neuronen | | | | |
|---|---|---|---|---|
| hinzugefügte Substanzen (µM) | | | Lebensfähigkeit der Zellen (% der Kontrolle) | |
| Aβ | Verbindung A | Verbindung B | | T-Test (Student) |
| 1h. 10 | | | | |
| 10 | - | - | 15±9,5 | - |
| 10 | 0,1 | - | 45±2,1 | * |
| 10 | - | 0,1 | 14±4,5 | |
| 10 | 1,0 | - | 59±14,5 | ** |
| 10 | - | 1,0 | 13±1,0 | |
| 10 | 2,5 | - | 66±4,5 | *** |
| 10 | - | 2,5 | 10±5,0 | |

| | | | | |
|---|---|---|---|---|
| *: P ≤ 0,01, | | | | |
| **: P ≤ 0,002, | | | | |
| *** P ≤ 0,0001, n=6 | | | | |

Der T-Test wurde an den Gruppen durchgeführt, denen das Aβ (10µM) zugesetzt wurde.

Wie der Tabelle 1 eindeutig zu entnehmen ist, wurde das Absterben der Neuronen von dem Aβ verursacht, wobei die Absterberate durch das Hinzufügen der Verbindung B nicht unterdrückt werden konnte. Wenn jedoch die Verbindung A in einer Menge von 0,1 µM, 1 µM oder 2,5 µM hinzugefügt wurde, konnte in allen Fällen das Neuronenabsterben signifikant unterdrückt werden (P ≤ 0,0082, P ≤ 0,0018 und P ≤ 0,0001).

### Beispiel 2

Kultivierte PC12-Zellen (die von Ratten abstammen, Chromaffiner Tumor) wurden in einer 100 mm Schale (von Corning hergestellt) unterkultiviert, die mit Polylysin beschichtet ist und die ein DMEM-Medium enthält, welches mit 10 % FCS and 5 % Pferdeserum (Produkt von Sigma) versetzt ist. Mit anderen Worten, die PC12-Zellen (1 x 10⁶ Zellen/Schale) wurden in einer mit Polylysin beschichteten 100 mm Schale angezüchtet. Um Unterschiede der PC12-Zellen aufzuzeigen, wuchsen die PC12-Zellen sieben Tage lang in einem DMEM-Medium, das 5 % FCS, 50 ng/ml NGF and 1 % Pferdeserum enthielt, heran. Die PC12-Zellen wurden dann durch Pipettierung abgeschert und in einem geeigneten Medium suspendiert, um so eine Zellsuspension zu erstellen. Die Zellsuspension wurde in jede Vertiefung einer mit Polylysin überzogenen 60 mm Schale mit 96 Vertiefungen (1 x 10⁴ Zellen/Vertiefung) gebettet. Nachdem die Adhäsion der Zellen bestätigt war, wurde das folgende Verfahren durchgeführt.

Die so anhaftenden PC12-Zellen wurden in einem DMEM-Medium angeordnet. 10 µM SNAP und 2,5 µM der Verbindung A wurden den Zellen zugesetzt. Bei einer Kontrollgruppe wurde nur SNAP hinzugefügt. Jede Probe wurde für 3 Stunden inkubiert. Hiernach wurde jede PC12-enthaltende Probe mit einer vorbereiteten DCFH-DA-Lösung (Molecular Probes, Inc.) in DMSO (1 mg/413 µl) versetzt, so daß eine Endkonzentration von 5 µM erzielt wurde. Die Proben wurden dann für 30 Minuten inkubiert. Die Zellen wurden unter Verwendung von Trypsin-EDTA entfernt und durch Zentrifugieren (1.000 rpm x 5 min.) wiedergewonnen. Die wiedergewonnenen Zellen wurden in 50 µl PBS(-) suspendiert, wobei sie mit Eis gekühlt wurden. Die Menge intrazellulären Wasserstoffperoxids wurde mit einem Durchflußzytometer bestimmt. Die Zellsuspension wurde einer FACS (Fluorescence-activated cell sorting) unterworfen und die Menge der durch Peroxide oxidierten, fluoreszierenden Substanz, 2',7'-Dichlorofluorescein (DCFH) wurde gemessen. Als Ergebnis wurde festgestellt, daß im Fall des Zusatzes von SNAP die Entstehung von Wasserstoffperoxid 1,3 - 1,4 mal höher war, als in dem Fall, in dem kein SNAP hinzugefügt wurde. Desweiteren wurde die durch den Zusatz von SNAP erhöhte Entstehung von Wasserstoffperoxid deutlich durch einen Zusatz von 2,5 µM der Verbindung A (P ≤ 0,0015) unterdrückt.

**Tabelle 2:**

| Wirkung der Verbindung A auf die unterdrückte Entstehung von Wasserstoffperoxid hervorgerufen durch NO (in den PC12-Zellen, die mit SNAP versetzt wurden) | | |
|---|---|---|
| behandelt mit | | Mengen der erzeugten H₂O₂ (% log Fluoreszenz der Kontrolle) |
| SNAP (µM) | Verbindung A (µM) | (*) |
| 0 | 0 | 100,0 |
| 10 | 0 | 135,8 ± 5,25 |
| 10 | 2,5 | 45,7 ± 6,03 |
| SNAP: S-Nitroso-N-Acetyl-DL-Penicillinamin | | |
| **: P ≤ 0,0015 [in Übereinstimmung mit dem Student T-Test in bezug auf SNAP (10 µM); n=3] | | |

Es ist berücksichtigt, daß das von dem Aβ abstammende NO die Entstehung von Wasserstoffperoxid beschleunigt. Wie jedoch der Tabelle 2 entnommen werden kann, wurde die Entstehung von Wasserstoffperoxid zufriedenstellend durch den Zusatz von 2.5 µM der Verbindung A unterdrückt.

Kürzlich wurde hervorgehoben, daß bei der Alzheimer-Krankheit die Apoptosis mit dem Neuronenabsterben in Zusammenhang stehen kann. Desweiteren wurde berichtet, daß das Neuronenabsterben durch das Aβ hervorgerufen wird und daß die Neurotoxizität durch die Entstehung von Wasserstoffperoxid verursacht wird (Proc. Natl. Acad. Sci. USA., 90:7951-7955, 1993).

Wie vorstehend beschrieben, wird die durch das Aβ induzierte Entstehung von Wasserstoffperoxid deutlich durch den Zusatz der Verbindung A unterdrückt. Daher konnte aufgezeigt werden, daß die Verwendung der Verbindung A exzellente präventive oder therapeutische Wirkungen auf die Alzheimer-Krankheit besitzt. Die in der vorliegenden Erfindung verwendete Verbindung A stellt somit eine besonders nützliche präventive oder therapeutische Zubereitung für die Alzheimer-Krankheit dar.

Die in dem vorliegenden Text verwendeten Abkürzungen bedeuten folgendes:
Polylysin ist ein Lysin-Polypetid mit variabler Kettenlänge;
DMEM bedeutet Delbecco's modified Eagle's medium;
DCFH ist 2',7'-Dichlorfluorescein;
FCS bedeutet fetal calf serum;
DMSO ist Dimethylsulfoxid;
Trypsin EDTA ist ein Komplexbildner auf der Basis von Äthylendiamintetraessigsäure und
NO ist Stickoxid.

## Patentansprüche

1. Verwendung von 2-Phenyl-1,2-Benzisoselenazol-3(2H)-one zur Herstellung einer pharmazeutischen Zubereitung zur Prophylaxe und/oder Therapie von Alzheimer-Krankheit.

2. Verwendung nach Anspruch 1, wobei das pharmazeutische Mittel als Tablette, Kapsel, Puder, Granulat, Sirup oder als Injektion darreichbar ist.

## Claims

1. The use of 2-phenyl-1,2-benzisoselenazol-3(2H)-one for producing a pharmaceutical preparation for the prophylaxis and/or therapy of Alzheimer's disease.

2. The use as claimed in claim 1 where the pharmaceutical composition can be administered as tablet, capsule, powder, granules, syrup or as injection.

## Revendications

1. Utilisation de la 2-phényl-1,2-benzisosélénazol-3(2H)-one pour la production d'une préparation pharmaceutique pour la prophylaxie et/ou la thérapie de la maladie d'Alzheimer.

2. Utilisation selon la revendication 1 où l'agent pharmaceutique peut être administré sous forme de comprimé, de capsule, de poudre, de granulés, de sirop ou d'injection.
